# EUROPEAN PATENT SPECIFICATION

(11) **EP 0 657 180 B1**
(45) Date of publication and mention of the grant of the patent: **17.11.1999**
(21) Application number: 94203560.1
(22) Date of filing: 07.12.1994
(51) Int. Cl.: A61M 5/162

(54) **Metering device**
Dosiervorrichtung
Dispositif de dosage

(30) Priority: 07.12.1993 NL 9302131
(43) Date of publication of application: 14.06.1995
(73) Proprietor: N.V. Nutricia, NL-2712 HM Zoetermeer (NL)
(72) Inventor: Kerkdijk, Joannes Lidvinus Aloysius Antonius, NL-2771 LK Boskoop (NL)
(74) Representative: Schumann, Bernard Herman Johan

(56) References cited:
- EP-A- 0 388 696
- FR-A- 1 301 522
- FR-E- 72 124
- US-A- 2 138 936
- US-A- 2 455 645

## Description

The invention relates to a metering device for liquid with medicinal application, for instance for dosing liquid food, intravenous liquids and the like, comprising a coupling piece which can be coupled to the drain opening of a liquid container, wherein an outflow aperture for liquid and a feed aperture for gas are arranged mutually separated in the coupling piece and wherein the feed aperture for gas is provided with a valve.

A metering device of the above stated type is known from US-A-2 455 645. Via a valve in the feed aperture for gas it is possible to supply gas to the liquid container at the moment that the pressure in the liquid container falls below a determined minimum value. A drawback to this metering device, particularly in the case liquid is administered intermittently, is that the liquid for dosing generally dries up in the vicinity of the valve as the aperture of the valve is in contact with the liquid in the container. Referred to here for instance is the administering at intervals of food and intravenous liquids. The liquid food can dry up in the vicinity of the valve whereby a good operation of the valve is obstructed and the flow of administered liquid decreases. Another drawback of the described device is possible occurrence of gas bubbles in the liquid for dosing.

The present invention has for its object to provide an improved device of the described type wherein the drawbacks of drying of liquid in the vicinity of the valve and the occurrence of gas bubbles in the liquid are limited.

The invention provides for this purpose a metering device wherein the valve aperture is enclosed by an air pocket formed by a screening cover and being in communication with the liquid via an orifice which is arranged in the wall of said cover and is smaller in size than said outflow aperture. Thus is achieved that the valve of the feed aperture for gas is wholly separated from the liquid whereby blockage and contamination thereof by drying of the liquid is prevented. The relatively small orifice in the wall of the cover forms a barrier against liquid flowing to the valve. The relatively small orifice has the additional advantage that no large gas bubbles will be formed in the liquid.

The relatively small orifice is preferably arranged in a side wall of the cover. In this way the danger is reduced of the relatively small orifice becoming blocked or dirtied during attachment of the metering device to a liquid container.

The side of the cover facing toward the liquid container is preferably provided with an acute angle. With this step the metering device can be coupled in simple manner to the liquid container. The outlet opening of the liquid container is usually covered prior to use with a thin layer of material. The acute angle of the housing enables the housing to penetrate through such a thin layer.

The invention will be further elucidated with reference to the non-limitative embodiments shown in the following figures, wherein:
fig. 1 shows a view of a metering device coupled to a liquid container;
fig. 2 is a cross section of a part of the device shown in fig. 1;

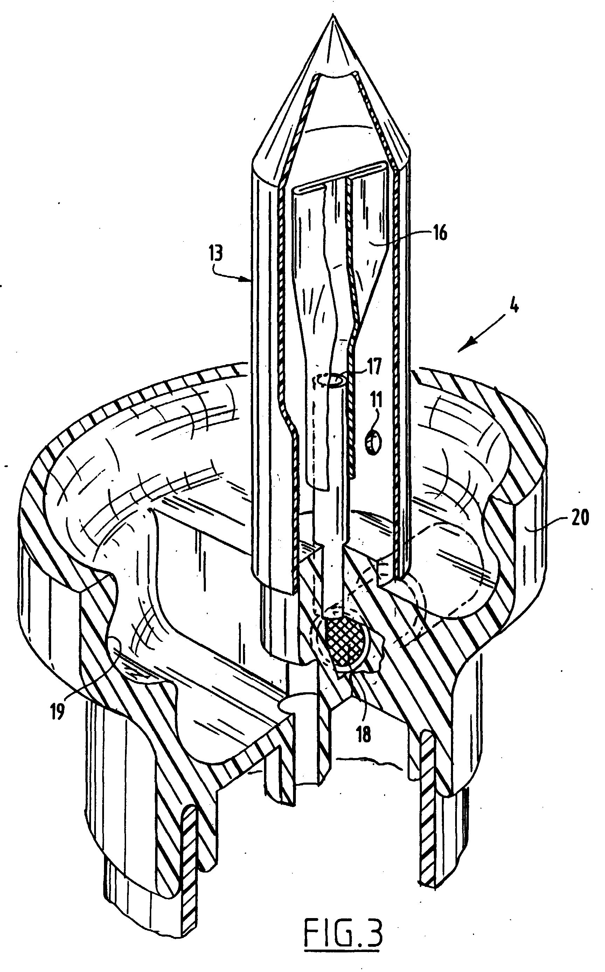

fig. 3 is a partly cut away perspective view of the upper part of a metering device similar to that of fig. 1 and 2; and
fig. 4 is a cross section of a part of an alternative device.

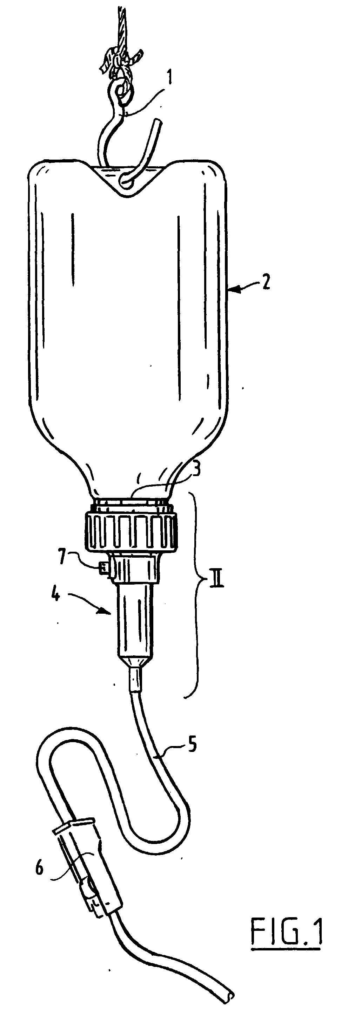

fig. 1 shows a liquid container 2 suspended from a hook 1 and having a drain opening 3 on the under-side thereof. A metering device 4 is coupled to the drain opening 3. By way of example the metering device 4 is provided in this figure with a flexible hose 5 with a control mechanism 6 for regulating the liquid flow through hose 5. This metering device 4 can be coupled to a catheter, such as a nose catheter or the like. It is also possible for a metering pump to be accommodated in the hose 5. Via an inlet 7 arranged on the side of the metering device 4 air can be supplied to liquid container 2.

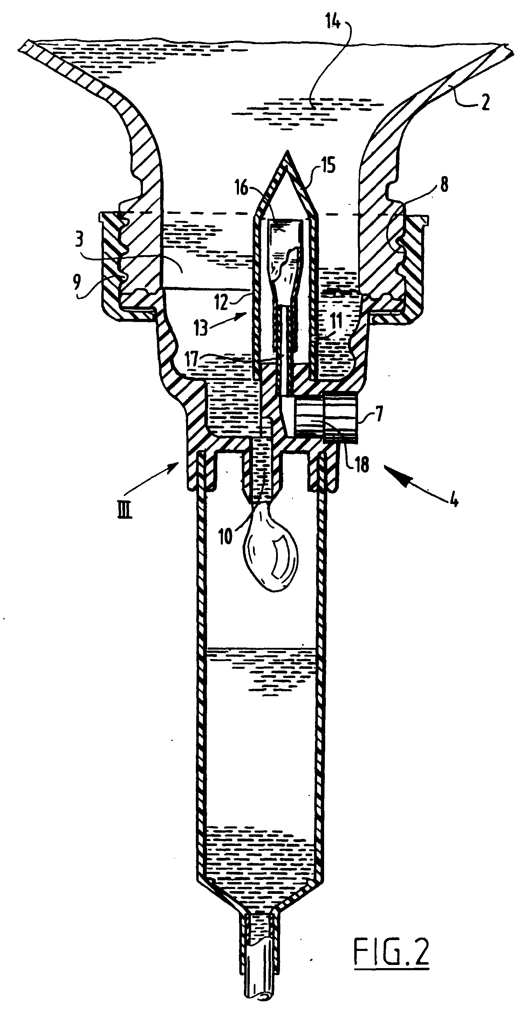

In fig. 2 the underside of liquid container 2 can be seen which is provided with an external screw thread 8. This can of course also be a snap-in or other connection. The metering device 4 is provided with an internal screw thread 9 whereby it can be connected in simple manner to the drain opening 3 of liquid container 2. An outflow aperture 10 for liquid arranged in metering device 4 is separated from a feed channel 17 for air. An orifice 11 is arranged in the side surface 12 of a cover 13. The orifice 11 is comparatively small relative to the outflow aperture 10. Due to the relatively small dimension of the orifice 11 liquid 14 is almost entirely prevented from reaching the inside of cover 13. The top part of the cover 13, that is, the side of the cover facing toward the liquid container 2, is provided with an acute angle 15. Situated on the inside of cover 13 is a pressure-sensitive valve 16, embodied here as diaphragm valve consisting of two mutually fixed layers of flexible material. Valve 16 can also be embodied otherwise, and conceivable here is for instance a ball valve. The valve 16 communicates with the inlet 7 by means of the feed channel 17. In order to prevent dirt present in the air from entering the liquid container 2 a filter 18 can be placed behind inlet 7. Said filter 18 can also be used for a sterile dosage of liquids. The metering device 4 can be made of sterilisable material.

In the partly cut away perspective view of the side of the metering device 4 facing toward the liquid channel container 2 the filter 18 communicating with the feed channel 17 can be clearly seen. At variance with the screw thread 8, 9 described along with fig. 2, the coupling means for coupling the metering device 4 to a liquid container 2 consist in this figure of an annular groove 19 in an engaging collar 20 manufactured from flexible material. The metering device 4 shown in fig. 2 also comprises a groove like said groove 9. The orifice 11 for air embodied in this figure as a circular hole may also have a different shape. A slot-like aperture could for instance be envisaged here. It is likewise possible to arranged a plurality of relatively small orifices in the cover 13.

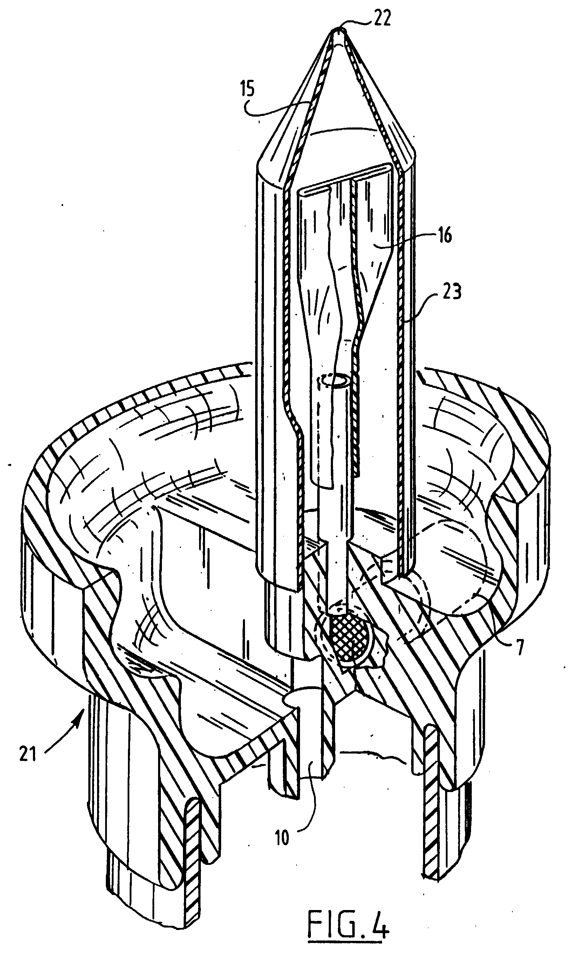

Fig. 4 shows an alternative metering device 21. This metering device 21 corresponds to a large extent with the metering device 4 as shown in fig. 2. The corresponding elements are designated with the same reference numerals as in fig. 2. At variance with the metering device 4 is the form of a screening cover 23 which encloses the pressure delivery valve 16. Cover 23 is provided with an orifice 22 for air which is situated at the top of cover 23, i.e. the side of cover 23 facing toward liquid container 2. The orifice 22 is situated at the position where the apex of the acute angle 15 was formerly situated.

## Claims

1. Metering device (4,21) for liquid with medicinal application for instance for dosing liquid food, intravenous liquids and the like, comprising a coupling piece which can be coupled to the drain opening (3) of a liquid container (2), wherein an outflow aperture (10) for liquid and a feed channel (17) for gas are arranged mutually separated in the coupling piece and wherein the feed channel (17) for gas is provided with a valve (16), **characterized in that** the valve aperture is enclosed by an air pocket formed by a screening cover (13, 23) and being in communication with the liquid via an orifice (11, 22) which is arranged in the wall of said cover (13, 23) and is smaller in size than said outflow aperture (10).

2. Metering device as claimed in claim 1, **characterized in that** the valve (16) is pressure-sensitive.

3. Metering device as claimed in claim 1 or 2, **characterized in that** the relatively small orifice (11) is arranged in a side wall (12) of the cover (13, 23).

4. Metering device as claimed in claim 3, **characterized in that** the side of the cover (13, 23) facing toward the liquid container (2) is provided with an acute angle (15).

## Patentansprüche

1. Dosiervorrichtung (4, 21) für Flüssigkeit zur medizinischen Anwendung, beispielsweise zum Dosieren von flüssiger Nahrung, intravenösen Flüssigkeiten und ähnlichem, mit einem Kupplungsteil, das mit der Auslaßöffnung (3) eines Flüssigkeitsbehälters (2) gekuppelt werden kann, wobei eine Ausströmöffnung (10) für Flüssigkeit und ein Zufuhrkanal (17) für Gas voneinander getrennt in dem Kupplungsteil angeordnet sind und wobei der Zufuhrkanal (17) für Gas mit einem Ventil (16) versehen ist, dadurch gekennzeichnet, daß die Ventilöffnung umhüllt bzw. umgeben ist von einer Lufttasche, die von einer abschirmenden Abdeckung (13, 23) gebildet ist und mit der Flüssigkeit in Verbindung steht über eine Öffnung (11, 22), die in der Wand der Abdeckung (13, 23) angeordnet und deren Größe geringer ist, als diejenige der Ausströmöffnung (10).

2. Dosiervorrichtung nach Anspruch 1, dadurch gekennzeichnet, daß das Ventil (16) druckempfindlich ist.

3. Dosiervorrichtung nach Anspruch 1 oder 2, dadurch gekennzeichnet, daß die relativ kleine Öffnung (11) in einer Seitenwand (12) der Abdeckung (13, 23) angeordnet ist.

4. Dosiervorrichtung nach Anspruch 3, dadurch gekennzeichnet, daß die in Richtung des Flüssigkeitsbehälters (2) weisende Seite der Abdeckung (13, 23) einen spitzen Winkel (15) aufweist.

## Revendications

1. Dispositif doseur (4, 21) pour un liquide ayant une application médicale, par exemple pour doser des aliments liquides, des liquides intraveineux et analogues, comprenant une pièce de couplage qui peut être couplée à l'ouverture d'évacuation (3) d'un conteneur de liquide (2), dans lequel une ouverture de décharge (10) pour le liquide et un canal d'alimentation (17) pour un gaz sont agencés de manière séparée l'une de l'autre dans la pièce de couplage et dans lequel le canal d'alimentation (17) pour le gaz est muni d'une vanne (16), **caractérisé en ce que** l'ouverture de vanne est entourée par une poche d'air formée par une enveloppe de blindage (13, 23) et qui est communication avec le liquide par l'intermédiaire d'un orifice (11, 22) qui est agencé dans la paroi de ladite enveloppe (13, 23) et est d'une taille plus petite que ladite ouverture de décharge (10).

2. Dispositif doseur selon la revendication 1, **caractérisé en ce que** la vanne (16) est sensible à la pression.

3. Dispositif doseur selon la revendication 1 ou 2, **caractérisé en ce que** l'orifice relativement petit (11) est agencé dans une paroi latérale (12) de l'enveloppe (13, 23).

4. Dispositif doseur selon la revendication 3, **caractérisé en ce que** le côté de l'enveloppe (13, 23) orienté vers le conteneur de liquide (2) comporte un angle aigu (15).
